(19) Europäisches Patentamt
European Patent Office
Office européen des brevets

(11) **EP 4 722 338 A1**

(12) **EUROPEAN PATENT APPLICATION**
published in accordance with Art. 153(4) EPC

(43) Date of publication:
08.04.2026 Bulletin 2026/15

(21) Application number: 24815598.8

(22) Date of filing: 31.05.2024

(51) International Patent Classification (IPC):
*C12N 5/00* (2006.01)          *C12N 1/00* (2006.01)
*C12P 21/00* (2006.01)

(52) Cooperative Patent Classification (CPC):
C12N 1/00; C12N 5/00; C12P 21/00

(86) International application number:
PCT/JP2024/019988

(87) International publication number:
WO 2024/248118 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.05.2023 JP 2023090288

(71) Applicant: FUJIFILM Corporation
Tokyo 106-8620 (JP)

(72) Inventors:
• INADA, Atsushi
Kanagawa 258-8577 (JP)
• NAKAI, Shinichi
Kanagawa 258-8577 (JP)
• SHIMONO, Katsuhiro
Kanagawa 258-8577 (JP)

(74) Representative: Hoffmann Eitle
Patent- und Rechtsanwälte PartmbB
Arabellastraße 30
81925 München (DE)

(54) **CELL CULTURE METHOD AND PRODUCT PRODUCING METHOD**

(57) An object of the present invention is to provide a cell culture method and a production method for a product, in which an occurrence of aggregates in foam is suppressed.

According to the present invention, there is provided a cell culture method of culturing cells at a cell density of $30 \times 10^6$ cells/mL or more and $400 \times 10^6$ cells/mL or less in a culture solution, the method including: step A of adjusting a poloxamer concentration in a foam liquid constituting foam to be 6 times or less a poloxamer concentration in the culture solution; or step B of adjusting a viscosity of the foam liquid constituting the foam to be 1.5 mPa·s or less under conditions of 35°C or higher and 38°C or lower.

EP 4 722 338 A1

## Description

## Technical Field

The present invention relates to a cell culture method and a production method for a product in which cells are cultured such that a poloxamer concentration of a foam liquid or a viscosity of the foam liquid satisfies a predetermined condition.

## Background Art

Cell culture is carried out for the intended purpose, for example, increasing the number of cells having useful properties or causing cells to produce a product. In high-density cell culture (for example, a cell density of $120 \times 10^6$ cells/mL), it is necessary to ensure sufficient oxygen supply to the cells. In general, kLa is known as an oxygen supply index, and kLa is also used as an oxygen supply index in scale-up. That is, it is desirable to perform design such that the same kLa as the kLa in a small scale can be realized even in a case of scale-up. For example, it is known that sufficient stirring power is applied as a unit for improving kLa. Meanwhile, the cells receive shear damage due to stirring. In particular, in a scale-up system, in a case where stirring speed is increased to increase the stirring power, shear due to rotation of a stirring blade is increased, and damage to the cells is increased. Examples of a unit for suppressing the shear damage to the cells due to stirring or the like include adding an additive having a cell protective effect to a culture solution (culture medium). A representative additive having a cell protective effect is a poloxamer (surfactant). In a case where a poloxamer concentration in the culture solution is low, the damage to the cells is increased.

Patent Document 1 discloses a method for recovering a recombinant protein, the method including: (1) a step of establishing cell culture by seeding mammalian cells expressing the recombinant protein in a bioreactor; (2) a step of perfusing a cell culture with a fresh cell culture medium formulated or supplemented to achieve a concentration of 1 g/L of a nonionic block copolymer and passing the cell culture through a hollow fiber filter having a pore diameter or a molecular weight cutoff that retains the recombinant protein in the bioreactor to maintain the cell culture and collect a permeate; (3) a step of, in a case where a predetermined parameter is reached, perfusing the cell culture with the fresh cell culture medium formulated or supplemented to achieve a concentration of 5 g/L of a nonionic block copolymer and passing the cell culture through a hollow fiber filter having a pore diameter or a molecular weight cutoff that does not retain the recombinant protein in the bioreactor; and (4) a step of collecting a permeate containing the recombinant protein.

Patent Document 2 discloses a cell culture method for cells, which includes a culture step of culturing a cell suspension having a cell density of $0.7 \times 10^8$ cells/mL or more in a culture tank while introducing a gas into a culture solution and a membrane separation treatment step of passing a cell suspension extracted from the culture tank through a separation membrane to separate the cell suspension into a cell-containing liquid and a permeated solution, where in the cell culture method for cells, an anti-foaming component to be added to the culture tank is such that an adding amount thereof per unit culture solution amount and per unit time is 0.70 mg/hour/L or less.

## Prior Art Documents

## Patent Documents

    Patent Document 1: JP7097404B
    Patent Document 2: WO2023/054556A

## Summary of Invention

## Object to be solved by the invention

It was found that, although foam is formed in an upper portion of the culture tank by gas aeration during culture, a poloxamer concentration in the foam was higher than the poloxamer concentration in the culture solution. Further, in a case where the poloxamer concentration in the foam is increased, cell-derived components (particularly, highly hydrophobic substances) in the foam aggregate, and aggregates are generated and accumulated. In a case where the aggregates in the foam fall into the culture solution, aggregates in the culture solution increase, and filtration membrane clogging in perfusion culture is promoted. As a result, it is not possible to maintain a desired culture period in continuous production. Therefore, it is important to suppress the increase in the poloxamer concentration in the foam.

An object to be achieved by the present invention is to provide a cell culture method and a production method for a product, in which an occurrence of aggregates in foam is suppressed.

**Means for solving the object**

As a result of diligent studies to achieve the above objects, the inventors of the present invention found that the above-described object can be achieved by culturing cells such that the poloxamer concentration in a foam liquid or a viscosity of the foam liquid satisfies a predetermined condition, whereby the present invention was completed.
That is, according to the present invention, the following inventions are provided.

<1> A cell culture method of culturing cells at a cell density of $30 \times 10^6$ cells/mL or more and $400 \times 10^6$ cells/mL or less in a culture solution, the method comprising:

step A of adjusting a poloxamer concentration in a foam liquid constituting foam to be 6 times or less a poloxamer concentration in the culture solution; or
step B of adjusting a viscosity of the foam liquid constituting the foam to be 1.5 mPa·s or less under conditions of 35°C or higher and 38°C or lower.

<2> The cell culture method according to <1>, in which step A is performed in a case where the poloxamer concentration in the foam liquid constituting the foam is 6 times or more the poloxamer concentration in the culture solution, or
step B is performed in a case where the viscosity of the foam liquid constituting the foam is 1.5 mPa·s or more under the conditions of 35°C or higher and 38°C or lower.
<3> The cell culture method according to <1> or <2>, in which the poloxamer concentration in the culture solution is 1.5 g/L or more and 15 g/L or less.
<4> The cell culture method according to any one of <1> to <3>, in which step A or step B includes a step of aerating the culture solution with a gas.
<5> The cell culture method according to <4>, in which, in a case where a cross-sectional area of a gas-liquid interface of a culture tank is denoted by S [cm$^2$], a total amount of a gas aeration amount into the culture solution is denoted by V [cm$^3$/min], and a height of a foam layer is denoted by H [cm], H, S, and V satisfy

$$H/(V/S) < 25 \text{ min.}$$

<6> The cell culture method according to <4> or <5>, in which, in a case where a cross-sectional area of a gas-liquid interface of a culture tank is denoted by S [cm$^2$] and a total amount of a gas aeration amount into the culture solution is denoted by V [cm$^3$/min], S and V satisfy

$$0.3 \text{ cm/min} < V/S < 9.4 \text{ cm/min.}$$

<7> The cell culture method according to any one of <1> to <6>, in which step A or step B includes a step of spraying, dropping, or scattering a liquid onto the foam.
<8> The cell culture method according to any one of <1> to <6>, in which step A or step B includes a step of removing the foam.
<9> The cell culture method according to <8>, in which the step of removing the foam is a step of rotating a stirring blade to eliminate the foam or incorporate the foam into the culture solution.
<10> A production method for a product, comprising: performing the cell culture method according to any one of <1> to <9>.
<11> The method according to <10>, in which the product is a protein.
<12> A production method for a product, comprising: culturing cells at a cell density of $30 \times 10^6$ cells/mL or more and $400 \times 10^6$ cells/mL or less in a culture solution, in which the method includes

step A of adjusting a poloxamer concentration in a foam liquid constituting foam to be 6 times or less a poloxamer concentration in the culture solution, or
step B of adjusting a viscosity of the foam liquid constituting the foam to be 1.5 mPa·s or less under conditions of 35°C or higher and 38°C or lower.

## Effect of the invention

According to the cell culture method and the production method for a product according to the present invention, the occurrence of the aggregates in the foam can be suppressed.

## BRIEF DESCRIPTION OF THE DRAWINGS

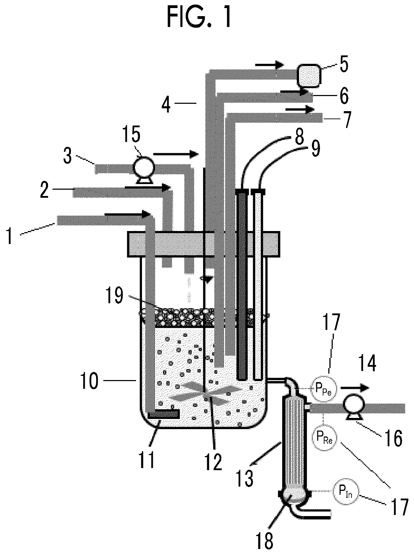

[FIG. 1] FIG. 1 shows a cell culture device.

## Embodiments for carrying out the invention

Hereinafter, the contents of the present invention will be described in detail. In the present specification, a numerical value range indicated using "to" means a range including numerical values described before and after "to" as a minimum value and a maximum value, respectively.

A cell culture method according to an embodiment of the present invention is a cell culture method of culturing cells at a cell density of $30 \times 10^6$ cells/mL or more and $400 \times 10^6$ cells/mL or less in a culture solution, the method including:

step A of adjusting a poloxamer concentration in a foam liquid constituting foam to be 6 times or less a poloxamer concentration in the culture solution, or

step B of adjusting a viscosity of the foam liquid constituting the foam to be 1.5 mPa·s or less under conditions of 35°C or higher and 38°C or lower.

The culture solution contains both cells and a culture supernatant.

In the present invention, the cell density of the culture solution is $30 \times 10^6$ cells/ml or more and $400 \times 10^6$ cells/ml or less, preferably $30 \times 10^6$ cells/ml or more and $300 \times 10^6$ cells/ml or less, more preferably $80 \times 10^6$ cells/mL or more and $240 \times 10^6$ cells/ml or less, still more preferably $80 \times 10^6$ cells/ml or more and $200 \times 10^6$ cells/ml or less, even still more preferably $80 \times 10^6$ cells/ml or more and $160 \times 10^6$ cells/ml or less, even still more preferably $100 \times 10^6$ cells/ml or more and $160 \times 10^6$ cells/ml or less, and particularly preferably $100 \times 10^6$ cells/ml or more and $120 \times 10^6$ cells/ml or less. M may be used to denote $10^6$.

The cell density can be measured by a live/dead cell analyzer Vi-CELL XR, manufactured by Beckman Coulter, Inc.

In the present invention, step A of adjusting the poloxamer concentration of the foam liquid constituting the foam to be 6 times or less the poloxamer concentration in the culture solution; or step B of adjusting the viscosity of the foam liquid constituting the foam to be 1.5 mPa·s or less under conditions of 35°C or higher and 38°C or lower is performed. Both step A and step B are intended to prevent an increase in the poloxamer concentration of the foam liquid constituting the foam.

The poloxamer concentration of the foam liquid and the poloxamer concentration in the culture solution can be measured by centrifuging the foam liquid or the culture solution at 300 G for 15 minutes, collecting the centrifugal supernatant liquid, and high performance liquid chromatography (HPLC).

The viscosity of the foam liquid can be measured by centrifuging the foam liquid at 300 G for 15 minutes, collecting the centrifugal supernatant liquid, heating the centrifugal supernatant liquid to 37°C in a water bath, and measuring the liquid viscosity with a viscometer. As the viscometer, for example, a vibration type viscometer (VM-10A-H manufactured by Sekonic Corporation) can be used.

Preferably, step A can be performed in a case where the poloxamer concentration of the foam liquid constituting the foam is 6 times or more the poloxamer concentration in the culture solution, or step B can be performed in a case where the viscosity of the foam liquid constituting the foam is 1.5 mPa·s or more under conditions of 35°C or higher and 38°C or lower. As a result, the poloxamer concentration of the foam liquid constituting the foam can be maintained at 6 times or less the poloxamer concentration in the culture solution, or the viscosity of the foam liquid constituting the foam can be maintained at 1.5 mPa·s or less under conditions of 35°C or higher and 38°C or lower.

The poloxamer concentration in the culture solution is preferably 1.5 g/L or more and 15 g/L or less. The poloxamer has a cell protective effect.

From the viewpoint of suppressing the aggregates, the poloxamer concentration in the culture solution is preferably 1.5 g/L or more and less than 6 g/L (more preferably 2.0 g/L or more and 5.8 g/L or less, still more preferably 3.0 g/L or more and 5.5 g/L or less, and even still more preferably 4.0 g/L or more and 5.5 g/L or less).

From the viewpoint of the cell protective effect, the poloxamer concentration in the culture solution is preferably 6 g/L or more and 15 g/L or less (more preferably 7 g/L or more and 14 g/L or less, still more preferably 8 g/L or more and 13 g/L or less, and even still more preferably 9 g/L or more and 12 g/L or less). In this case, the amount of the aggregates increases, but it is effectively suppressed by the present invention.

Step A or step B preferably includes a step of aerating the culture solution with a gas.

In a case where a cross-sectional area of a gas-liquid interface of a culture tank is denoted by S [$cm^2$], a total amount of a gas aeration amount into the culture solution is denoted by V [$cm^3$/min], and a height of a foam layer is denoted by H [cm], H, S, and V preferably satisfy

$$H/(V/S) < 25 \text{ min},$$

more preferably satisfy

$$H/(V/S) < 20 \text{ min},$$

still more preferably satisfy

$$H/(V/S) < 16 \text{ min},$$

and
particularly preferably satisfy

$$H/(V/S) < 10 \text{ min}.$$

The lower limit of H/(V/S) is not particularly limited and is generally 1 min or more.

In a case where a cross-sectional area of a gas-liquid interface of a culture tank is denoted by S [$cm^2$] and a total amount of a gas aeration amount into the culture solution is denoted by V [$cm^3$/min], S and V preferably satisfy

$$0.3 \text{ cm/min} < V/S < 9.4 \text{ cm/min},$$

more preferably satisfy

$$1.0 \text{ cm/min} < V/S < 9.4 \text{ cm/min},$$

and
still more preferably satisfy

$$2.9 \text{ cm/min} < V/S < 9.4 \text{ cm/min}.$$

The cross-sectional area S [$cm^2$] of the gas-liquid interface of the culture tank can be calculated based on the inner diameter of the culture tank.

The total amount V [$cm^3$/min] of the gas aeration amount into the culture solution is based on the set amount of the gas aeration amount in the gas supply unit.

The height H [cm] of the foam layer is an average value in the culture tank cross section. The average value is obtained by measuring the heights of any three points and averaging the three points.

Step A or step B may include a step of spraying, dropping, or scattering a liquid onto the foam. By spraying, dropping, or scattering a liquid onto the foam, the poloxamer concentration in the foam liquid constituting the foam or the viscosity of the foam liquid constituting the foam can be reduced. In a case of spraying, dropping, or scattering the liquid, the liquid may be sprayed, dropped, or scattered from the upper portion of the culture tank, but the present invention is not particularly limited thereto.

Step A or step B may include a step of removing foam. By removing the foam, the poloxamer concentration in the foam liquid constituting the foam or the viscosity of the foam liquid constituting the foam can be reduced.

As the step of removing the foam, for example, a step of rotating a stirring blade to eliminate the foam or incorporate the foam into the culture solution may be used, but the present invention is not particularly limited thereto. It is preferable that the stirring blade is provided at a position that comes into contact with the foam. Specifically, the stirring blade can be provided in the culture container such that the stirring blade is located near the uppermost portion of the culture solution.

In the cell culture method according to the embodiment of the present invention, it is preferable that the culture solution containing the cells is extracted (bled) from the culture tank such that the cell density is maintained at a predetermined cell density after the cell density reaches the predetermined cell density.

The bleeding is sufficient to be such that the culture solution containing cells can be extracted from the culture container, and the method thereof is not particularly limited. However, for example, as shown in FIG. 1, a pipe for discharging the culture solution is inserted into the culture solution, and the culture solution can be discharged from the culture container through the pipe.

Preferably, in the bleeding, the culture solution may be bled while an electrostatic capacity of the culture solution is subjected to in-line measurement. The in-line measurement is to measure any measurement value indicating a state of the culture solution while carrying out cell culture. In order to subject the electrostatic capacity to in-line measurement, for example, as shown in FIG. 1, it is sufficient to insert an electrostatic capacity sensor into the culture solution and subject the electrostatic capacity of the culture solution to in-line measurement while carrying out culture.

Preferably, the bleeding may be automatically controlled. The automatic control means that the start or stop of the bleeding is automatically controlled based on any measurement value indicating any state of the culture solution. For example, the start and stop of the bleeding can be automatically controlled based on the measurement value of the electrostatic capacity of the culture solution.

Preferably, the bleeding may be carried out such that a variation of the electrostatic capacity of the culture solution, where the electrostatic capacity is subjected to in-line measurement, is $\pm 20\%$ or less with respect to an average value of an electrostatic capacity during the production period. The variation of the electrostatic capacity of the culture solution, where the electrostatic capacity is subjected to in-line measurement, is more preferably $\pm 15\%$ or less with respect to an average value of an electrostatic capacity during the production period, still more preferably $\pm 12\%$ or less with respect to the average value of the electrostatic capacity during the production period, and particularly preferably $\pm 8\%$ or less with respect to the average value of the electrostatic capacity during the production period. The lower limit of the variation of the electrostatic capacity of the culture solution is not particularly limited, and it is sufficient to be 0% or more; however, a variation of substantially 0.1% or more occurs in consideration of the variation of daily cell proliferation.

One exemplary bleeding can be carried out by a step including:

(i) setting an electrostatic capacity serving as a target;
(ii) setting a weight control value of the culture container;
(iii) measuring the electrostatic capacity of the culture solution at a period of 0.1 seconds or less;
(iv) driving a pump for draining the culture solution at a rate of 0.3 vvd to 3 vvd in a case where the electrostatic capacity of the culture solution exceeds the target (here, vvd means "volume of drained culture solution/volume of culture solution/day");
(v) automatically supplying a culture medium to the culture container while measuring a mass of the culture solution such that a variation of a liquid amount of the culture solution is within 10%; and
(vi) stopping the pump for draining the culture solution in a case where the electrostatic capacity of the culture solution is smaller than the target by 0.01% or more.

FIG. 1 shows one example of a cell culture device that can be used in the cell culture in the present invention. In FIG. 1, a culture container 10 is a container that accommodates a culture solution containing cells. Cells are cultured in the culture solution in the inside of the culture container 10.

Oxygen and/or air are sent from a sparger air supply pipe 1, and the oxygen and/or the air are introduced into the culture solution through a sparger 11 having a hole diameter of 20 $\mu$m. The dissolved oxygen concentration inside the culture solution can be adjusted by the sparger 11. The sparger is not particularly limited; however, it is possible to use, for example, a sparger in which an average hole diameter of a gas release unit is 1 $\mu$m or more and 300 $\mu$m or less and which releases a gas containing 30% by volume or more of oxygen.

Air and/or carbon dioxide are introduced from an air supply pipe 2 to an upper part of the culture solution in the culture container.

The culture medium is supplied to the culture container from a culture medium supply pipe 3. A culture medium supply pump 15 is provided in the culture medium supply pipe 3.

A gas exhaust pipe 4 is a pipe for gas exhaust, and a gas exhaust filter 5 is connected to a terminal of the gas exhaust pipe 4.

A sampling tube 6 is a pipe for collecting (sampling) the culture solution.

A bleeding tube 7 is a pipe for extracting (bleeding) the culture solution.

An electrostatic capacity sensor 8 is mounted to come into contact with the culture solution in the culture container.

A dissolved oxygen sensor 9 is mounted to come into contact with the culture solution in the culture container.

A stirring member having a stirring blade 12 may be provided inside the culture container 10. In a case where the stirring blade 12 is rotated, the culture solution inside the culture container 10 is stirred, and the homogeneity of the culture solution is maintained. In a case where the culture solution is stirred by the stirring blade 12, the bubbles released by the sparger are also stirred. The position of the stirring member having a stirring blade, the size of the stirring blade, and the like are not particularly limited and may be designed depending on the cell kind to be used, the amount of the culture solution, the amount of oxygen to be supplied, or the position, number, size, or the like of the sparger. Further, in order to quickly stir bubbles coming out of the sparger and suppress coalescence of the bubbles, it is preferable to dispose the stirring blade 12 at a position close to the sparger. FIG. 1 shows a state in which foam 19 is present.

A perfusion device 13 is connected to a lower part of the culture container 10. In addition, the culture solution inside the culture container 10 passes through the perfusion device 13 (hollow fiber MF membrane), and the permeated solution containing the cell product is extracted from the culture container 10 by a delivery pump 16. The flow of the permeated solution is indicated by an arrow 14. A pressure gauge 17 and a diaphragm pump 18 may be installed in the perfusion device 13.

In the present invention, a cell suspension extracted from the culture container may be allowed to pass through a separation membrane to separate the cell suspension into a cell-containing liquid and a permeated solution. This operation can be carried out using a perfusion device. In this operation, the cell suspension extracted from the culture container is separated into a cell-containing liquid having a cell density higher than that of the cell suspension and a permeated solution having a cell density lower than that of the cell suspension. The cell density can be measured by a live/dead cell analyzer Vi-CELL XR, manufactured by Beckman Coulter, Inc.

The membrane separation treatment step described above is preferably tangential filtration, more preferably alternating tangential flow (ATF) or tangential flow, and most preferably alternating tangential flow. Examples of the filter capable of carrying out the alternating tangential flow include SuATF10-S02PES or F2 RF02PES, manufactured by Repligen Corporation.

As the culture medium that is used for cell culture, a culture medium that is generally used for culturing animal cells can be used. For example, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

Although the pH of the culture medium varies depending on the cells to be cultured, the culture medium generally has a pH of 6.0 to 8.0, preferably has a pH of 6.4 to 7.6, and more preferably has a pH of 6.7 to 7.4.

The culture temperature is generally 30°C to 40°C, preferably 32°C to 39°C, and more preferably 36°C to 38°C, and the culture temperature may be changed during the culture.

The culture can be carried out in an atmosphere having a $CO_2$ concentration of 0% to 40% by volume, preferably 2 to 25% by volume, and more preferably 3 to 20% by volume.

The amount of the culture solution is preferably 1 L or more, more preferably 50 L or more, and still more preferably 200 L or more.

The culture period is not particularly limited, and is the same as the period of the perfusion culture described later. In the present invention, the culture period is preferably 14 days or more.

In the culture, the culture medium can be replaced, aerated, and stirred as necessary. In a case of carrying out stirring of the culture, the rotation speed of stirring is not particularly limited; however, the stirring power per unit volume is generally 10 to 300 $kW/m^3$, preferably 20 to 200 $kW/m^3$, and more preferably 30 to 100 $kW/m^3$.

The dissolved oxygen concentration in the culture solution can be appropriately set and is not particularly limited; however, it is 20% to 150%, preferably 30% to 120%, and more preferably 30% to 100% in a case where the saturated dissolved oxygen concentration in the liquid at 37°C in air at 1 atm is set to 100%.

The cell culture can be carried out using a cell culture device having the configuration described above in the present specification. The cell culture device may be any one of a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like. The culture container is preferably a single-use culture tank from the viewpoint of homogenizing the culture environment or the like. From the viewpoint of homogenization of the culture environment and the like, the cell culture device may be a single-use culture tank.

The viscosity of the culture solution is preferably 1.2 mPa·s or more and less than 15 mPa·s, more preferably 1.4 mPa·s or more and less than 12 mPa·s, and particularly preferably 1.6 mPa·s or more and less than 10 mPa·s.

In the present invention, a pH adjusting agent may be added during the culture. From the viewpoint of the clogging of the filtration filter flow channel, a pH adjusting agent to be added to the culture tank is such that an adding amount thereof per day is preferably 8 mmol/day/L or less and more preferably 7 mmol/day/L or less. Here, it is not necessary to add a pH adjusting agent.

The pH adjusting agent is not particularly limited; however, it is preferably an aqueous $Na_2CO_3$ solution, an aqueous NaOH solution, or an aqueous $NaHCO_3$ solution, and it is more preferably an aqueous $NaHCO_3$ solution. The pH adjusting agent may be added alone or may be added by being mixed with a culture medium or an anti-foaming agent. In order to avoid the local variation of pH in the culture solution due to the addition of the pH adjusting agent, it is preferable that the pH adjusting agent is added by being mixed with a culture medium.

In the present invention, the mode of the method of culturing cells is preferably perfusion culture.

The perfusion culture is a culture method in which a fresh culture medium is added and at the same time the used culture medium is removed. In general, the perfusion culture makes it possible to achieve a high viable cell density. A typical perfusion culture begins with a batch culture start-up lasting 1 or 2 days, thereafter a fresh supply culture medium is added to the culture continuously, stepwise, and/or intermittently, and the used culture medium is removed at the same time. In the perfusion culture, methods such as sedimentation, centrifugation, and filtration can be used to remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture and the used culture medium that is removed may substantially not include cells or may have much fewer cells than the culture. A product that is expressed by cell culture can be retained in the culture or recovered by the selection of the membrane pore diameter.

The period of the perfusion culture is preferably 5 days or more and 150 days or less, more preferably 10 days or more and 100 days or less, and still more preferably 20 days or more and 90 days or less. It is preferable for the number of days of the culture to be 5 days or more since the obtained cell number is large and the production amount of a product is large in a case where the cells produce the product. It is preferable for the number of days of the culture to be 150 days or less from the viewpoint of preventing clogging of the filtration membrane used in the perfusion culture and preventing contamination.

The perfusion ratio is not particularly limited; however, it is generally 0.3 vvd to 5.0 vvd, preferably 0.5 vvd to 3.0 vvd, and more preferably 0.8 vvd to 2.5 vvd. The vvd regarding the perfusion ratio means "volume of drained culture solution/volume of culture solution in culture container/day". It is noted that the perfusion ratio described herein is a parameter different from "(iv) driving a pump for draining the culture solution at a rate of 0.3 vvd to 3 vvd in a case where the electrostatic capacity of the culture solution exceeds the target" in one example of the bleeding.

The extraction of the culture solution from the culture container can usually be carried out using a pump, but other available liquid feeding units may be used. The culture solution extracted from the culture container is subjected to treatments such as product recovery and removal of the dead cells. The culture solution extracted from the culture container may be partially discarded or returned to the culture container after treatments such as product recovery and removal of the dead cells. In a case where a loss of the culture medium occurs due to the above treatments, the loss can be compensated, for example, by supplying a fresh culture medium to the culture container.

The kind of the cell in the present invention is not particularly limited; however, examples thereof include eukaryotic cells such as an animal cell, a plant cell, and yeast, prokaryotic cells such as Bacillus subtilis, and Escherichia coli. The cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a C127 cell, a myeloma cell (such as an NS0 cell), a PerC6 cell, an SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. The cell may be a stem cell such as an embryonic stem cell (ES cell) or an induced pluripotent stem cell (iPS cell). Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 80% or more, more preferably 85% or more, particularly preferably 90% or more, and most preferably 95% or more.

These cells may be cells into which a foreign gene encoding a protein desired to be expressed (for example, an antibody) has been introduced. The cell is preferably a cell that produces an antibody. An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of the cytomegalovirus (CMV), an early promoter of SV40, a retrovirus promoter, a metallothionein promoter, a heat shock promoter, an SRα promoter, and a promoter and enhancer of the Moloney murine leukemia virus. In addition, an enhancer of the IE gene of human CMV may be used together with the promoter.

As the selection marker gene, it is possible to use, for example, a drug resistance gene (a neomycin resistance gene, a DHFR gene, a puromycin resistance gene, a blasticidin resistance gene, a hygromycin resistance gene, a cycloheximide resistance gene), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

The production method for a product according to the embodiment of the present invention is a method including performing the cell culture method according to the embodiment of the present invention. In the production method for a product according to the embodiment of the present invention, preferably, a cell is cultured using a cell culture device to produce a product from the cell.

That is, according to the present invention, there is provided a production method for a product, including: culturing cells at a cell density of $30 \times 10^6$ cells/mL or more and $400 \times 10^6$ cells/mL or less in a culture solution, in which the method includes

step A of adjusting a poloxamer concentration in a foam liquid constituting foam to be 6 times or less a poloxamer concentration in the culture solution, or
step B of adjusting a viscosity of the foam liquid constituting the foam to be 1.5 mPa·s or less under conditions of 35°C or higher and 38°C or lower.

According to the present invention, there is provided a product that is produced by the production method for a product according to the embodiment of the present invention.

In the present invention, the kind of product is not particularly limited; however, the product is preferably a protein. Examples of the product include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv). In addition, it may be, in addition to the above, an adenovirus, an adeno-associated virus, a lentivirus, or the like.

The product is preferably an antibody, and more preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culturing by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other, and the like. In addition, a bispecific antibody can be expressed by introducing a gene encoding the bispecific antibody into a cell.

The Fc fusion protein indicates a protein having an Fc region and includes an antibody.

The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

Regarding the product recovery, the culture solution may be simply recovered. For example, a liquid obtained by removing at least a part of the cells from the culture solution using a filter or a centrifuge may be recovered, and a known method is used without particular limitation. In a case of desiring to improve the purity of the product, change the solvent of the product, or change the form of the product to, for example, a powder form, the culture solution or the liquid can be subjected to further treatment.

In addition, a part of the culture solution can be recovered while being perfused, or a part of the culture solution can be recovered as a liquid which is obtained by removing at least a part of cells from the part of the culture solution, which is recovered by using a filter or a centrifuge while being perfused.

The product can be purified by a purification treatment. The obtained product can be purified to high purity. For the separation and purification of the product, the separation and purification methods that are used for general proteins may be used. For example, it is possible to carry out separation and purification of a product by appropriately selecting and combining means such as a chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the product obtained as above can be measured according to absorbance measurement, enzyme-linked immunosorbent assay (ELISA), or the like. In addition, in a case where the product is an antibody, the titer of the antibody can also be measured with a commercially available analytical instrument such as Cedex Bio manufactured by F. Hoffmann-La Roche, Ltd.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify the product or partially remove a peptide thereof by allowing an appropriate polypeptide modifying enzyme to act on the useful substance before or after purification. As the polypeptide modifying enzyme, for example, trypsin, chymotrypsin, lysyl endopeptidase, protein kinase, or glucosidase is used.

The product that is produced according to the present invention can be used, for example, for a biopharmaceutical product, or regenerative medicine.

The present invention will be more specifically described using the following examples; however, it is not limited by the examples.

Examples

<Establishment of antibody-producing cell>

A vector containing a nucleic acid sequence encoding IgG1 was constructed, and the constructed vector was introduced into CHO-DG44 cells, whereby CHO-DG44 cells expressing IgG1 (IgG1 cells) were prepared. The construction of the vector and the introduction thereof into the cell were carried out according to Example 2 of JP2016-517691A. As described above, CHO cells producing monoclonal antibodies were prepared and used in the following experiment.

<Cell culture>

In Examples and Comparative Examples, experiments were carried out using the cell culture device shown in FIG. 1. The culture medium was adjusted with reference to the exchange culture medium in Table 2 of JP2000-517188A.

A culture medium (0.83 L) was charged into a glass culture tank (cross-sectional area of 102 cm$^2$) having a

diameter of 11.4 cm.

A culture medium (7.1 L) was charged into a glass culture tank (cross-sectional area of 398 cm$^2$) having a diameter of 22.5 cm.

A culture medium (50 L) was charged into a plastic culture tank (cross-sectional area of 957 cm$^2$) having a diameter of 34.9 cm.

CHO cells were seeded in the above culture medium at $0.5 \times 10^6$ cells/mL.

The rotation speed of stirring was 180 rpm with a paddle having a diameter of 85 mm in a case of a cross-sectional area of 102 cm$^2$, 99 rpm with a paddle having a diameter of 154 mm in a case of a cross-sectional area of 398 cm$^2$, and 250 rpm with a propeller having a diameter of 111 mm in a case of a cross-sectional area of 957 cm$^2$. From the upper surface, air and $CO_2$ were mixed so that the $CO_2$ concentration was 5% and a total aeration amount of 0.01 vvm was supplied , and from the bottom surface, $O_2$ was automatically controlled and supplied from a sparger installed in the culture tank so that the oxygen concentration in the culture solution was 80%.

After culturing for 2 days after seeding, the cell culture solution was continuously filtered with ATF2 manufactured by Repligen Corporation in a case of a cross-sectional area of 102 cm$^2$ and a cross-sectional area of 398 cm$^2$ and with ATF4 manufactured by Repligen Corporation in a case of a cross-sectional area of 957 cm$^2$, and the recovery liquid was recovered while continuously supplying a culture medium at a perfusion ratio of 0.6 vvd.

Further, on the 5th day, the perfusion ratio was changed to 1.2 vvd.

Thereafter, in a case where the cell density reached the cell density shown in Table 1, the cell suspension was extracted from the culture tank (bleeding), and the same amount of a fresh culture medium was supplemented at a frequency of at least once a day or continuously so that the cell density is maintained at the cell density shown in Table 1, whereby the viable cell density was maintained while a predetermined culture solution amount being maintained. The period from the start of the culture to the first start of bleeding was defined as the cell proliferation period, and the period after the first start of bleeding was defined as the antibody production period.

The culture conditions to be used in this case were set as shown in Table 1 to carry out Examples 1 to 9 and Comparative Examples 1 to 4.

Thereafter, the culture was continued for 10 days or more, and the evaluation described below was carried out.

<Automatic control method for cell bleeding>

In a case where the cell bleeding was automatically controlled, the cell bleeding was continuously carried out automatically using a FUTURA sensor manufactured by ABER Instruments Ltd. while monitoring the electrostatic capacity of the culture solution. Specifically, the measurement was carried out by the following method.

(1) An electrostatic capacity serving as a target is set.
(2) A weight control value of the culture tank is set.
(3) The electrostatic capacity of the culture solution is measured at a period of 0.1 s or less.
(4) A pump for draining the culture solution is driven at a rate of 0.3 vvd to 3 vvd in a case where the electrostatic capacity of the culture solution exceeds the target.
(5) A culture medium is automatically supplied into the culture tank while the weight of the culture tank is measured so that the liquid amount of the culture solution is roughly kept constant.
(6) The pump for draining the culture solution is stopped in a case where the electrostatic capacity of the culture solution is smaller than the target by 0.01% or more.

[Measurement and evaluation method]

<Method of collecting foam liquid>

The foam is withdrawn from the sterilized tube installed in the culture tank, and the liquefied foam is used as the foam liquid. In a case where the height of the foam layer is 6 cm or more, the foam liquid in the culture tank is collected from a position approximately 3 cm below the uppermost portion of the foam layer. In a case where the height of the foam layer is less than 6 cm, the foam liquid is collected from substantially the central portion of the height of the foam layer (central portion of the culture solution surface and the foam layer surface).

<Measuring method of poloxamer concentration in foam liquid>

The foam liquid is centrifuged at 300 G for 15 minutes, the centrifugal supernatant liquid is collected, and the concentration of the poloxamer contained therein is measured by HPLC.

<Measuring method of viscosity of foam liquid>

The foam liquid is centrifuged at 300 G for 15 minutes, the centrifugal supernatant liquid is collected, the centrifugal supernatant liquid is heated to 37°C in a water bath, and the liquid viscosity is measured with a viscometer. As the viscometer, for example, a vibration type viscometer (VM-10A-H manufactured by Sekonic Corporation) is used.

<Determination of presence or absence of aggregates>

The aggregates are visually inspected in the foam liquid. The collected foam liquid was allowed to stand, and the presence or absence of the aggregates was determined based on whether or not the precipitation occurred.
Table 1 shows measurement results of Examples and Comparative Examples.

[Table 1]

| | Cell density | Poloxamer concentration in culture solution | Concentration ratio of poloxamer | Viscosity | Cross-sectional area of a gas-liquid interface of culture tank (S) | Gas aeration amount (V) | Height of foam (H) | V/S | H/(V/S) | Presence or absence of aggregates |
|---|---|---|---|---|---|---|---|---|---|---|
| | M cells/mL | g/L | times | mPa·s | cm$^2$ | cm$^3$/min | cm | cm/min | min | - |
| Example 1 | 120 | 5 | 1.5 | 1 | 102 | 33.2 | 3 | 0.3 | 9.2 | Absent |
| Example 2 | 120 | 5 | 6.0 | 1.5 | 398 | 213 | 13 | 0.5 | 24.3 | Absent |
| Example 3 | 120 | 5 | 2.4 | 1.1 | 398 | 1136 | 13 | 2.9 | 4.6 | Absent |
| Example 4 | 120 | 5 | 2.2 | 1.1 | 957 | 9000 | 29.5 | 9.4 | 3.1 | Absent |
| Example 5 | 120 | 10 | 2.1 | 1.2 | 398 | 1136 | 13 | 2.9 | 4.6 | Absent |
| Example 6 | 120 | 10 | 1.9 | 1.2 | 957 | 7200 | 29.5 | 7.5 | 3.9 | Absent |
| Example 7 | 120 | 10 | 2.9 | 1.4 | 102 | 33.2 | 4 | 0.3 | 12.3 | Absent |
| Example 8 | 80 | 5 | 4.0 | 1.2 | 957 | 450 | 7.5 | 0.5 | 15.9 | Absent |
| Example 9 | 200 | 5 | 2.1 | 1.1 | 102 | 66.4 | 8.1 | 0.7 | 12.5 | Absent |
| Comparative Example 1 | 120 | 5 | 7.0 | 1.6 | 102 | 24.9 | 7.5 | 0.2 | 30.7 | Present |
| Comparative Example 2 | 120 | 5 | 14.0 | 2.1 | 398 | 284 | 21 | 0.7 | 29.4 | Present |
| Comparative Example 3 | 120 | 5 | 8.5 | 1.6 | 957 | 900 | 25 | 0.9 | 26.6 | Present |
| Comparative Example 4 | 120 | 10 | 9.5 | 2.5 | 398 | 284 | 21 | 0.7 | 29.4 | Present |

Explanation of References

1: sparger air supply pipe

2: air supply pipe

3: culture medium supply pipe

4: gas exhaust pipe

5: gas exhaust filter

6: sampling tube

7: bleeding tube

8: electrostatic capacity sensor

9: dissolved oxygen sensor

10: culture container

11: sparger

12: stirring blade

13: perfusion device

14: flow of permeated solution

15: culture medium supply pump

16: delivery pump

17: pressure gauge

18: diaphragm pump

19: foam

## Claims

1.  A cell culture method of culturing cells at a cell density of $30 \times 10^6$ cells/mL or more and $400 \times 10^6$ cells/mL or less in a culture solution, the method comprising:

    step A of adjusting a poloxamer concentration in a foam liquid constituting foam to be 6 times or less a poloxamer concentration in the culture solution, or
    step B of adjusting a viscosity of the foam liquid constituting the foam to be 1.5 mPa·s or less under conditions of 35°C or higher and 38°C or lower.

2.  The cell culture method according to claim 1,

    wherein step A is performed in a case where the poloxamer concentration in the foam liquid constituting the foam is 6 times or more the poloxamer concentration in the culture solution, or
    step B is performed in a case where the viscosity of the foam liquid constituting the foam is 1.5 mPa·s or more under the conditions of 35°C or higher and 38°C or lower.

3. The cell culture method according to claim 1,
wherein the poloxamer concentration in the culture solution is 1.5 g/L or more and 15 g/L or less.

4. The cell culture method according to claim 1,
wherein step A or step B includes a step of aerating the culture solution with a gas.

5. The cell culture method according to claim 4,
wherein, in a case where a cross-sectional area of a gas-liquid interface of a culture tank is denoted by S [cm$^2$], a total amount of a gas aeration amount into the culture solution is denoted by V [cm$^3$/min], and a height of a foam layer is denoted by H [cm], H, S, and V satisfy

$$H/(V/S) < 25 \text{ min},$$

6. The cell culture method according to claim 4,
wherein, in a case where a cross-sectional area of a gas-liquid interface of a culture tank is denoted by S [cm$^2$] and a total amount of a gas aeration amount into the culture solution is denoted by V [cm$^3$/min], S and V satisfy

$$0.3 \text{ cm/min} < V/S < 9.4 \text{ cm/min},$$

7. The cell culture method according to any one of claims 1 to 6,
wherein step A or step B includes a step of spraying, dropping, or scattering a liquid onto the foam.

8. The cell culture method according to any one of claims 1 to 6,
wherein step A or step B includes a step of removing the foam.

9. The cell culture method according to claim 8,
wherein the step of removing the foam is a step of rotating a stirring blade to eliminate the foam or incorporate the foam into the culture solution.

10. A production method for a product, comprising:
performing the cell culture method according to any one of claims 1 to 6.

11. The method according to claim 10,
wherein the product is a protein.

12. A production method for a product, comprising:

culturing cells at a cell density of $30 \times 10^6$ cells/mL or more and $400 \times 10^6$ cells/mL or less in a culture solution, wherein the method includes
step A of adjusting a poloxamer concentration in a foam liquid constituting foam to be 6 times or less a poloxamer concentration in the culture solution, or
step B of adjusting a viscosity of the foam liquid constituting the foam to be 1.5 mPa·s or less under conditions of 35°C or higher and 38°C or lower.

# FIG. 1

## INTERNATIONAL SEARCH REPORT

| | International application No. |
|---|---|
| | **PCT/JP2024/019988** |

**A. CLASSIFICATION OF SUBJECT MATTER**

*C12N 5/00*(2006.01)i; *C12N 1/00*(2006.01)i; *C12P 21/00*(2006.01)i
FI: C12N5/00; C12P21/00 A; C12N1/00 B

According to International Patent Classification (IPC) or to both national classification and IPC

**B. FIELDS SEARCHED**

Minimum documentation searched (classification system followed by classification symbols)

C12N5/00; C12N1/00; C12P21/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2023/054556 A1 (FUJIFILM CORPORATION) 06 April 2023 (2023-04-06) claims 1-2, 8, 17-18, paragraphs [0009], [0036], [0040], [0044], examples | 1-12 |
| A | WO 2022/084386 A1 (MERCK PATENT GMBH) 28 April 2022 (2022-04-28) | 1-12 |
| A | WO 2020/003833 A1 (FUJIFILM CORPORATION) 02 January 2020 (2020-01-02) | 1-12 |

☐ Further documents are listed in the continuation of Box C.   ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **05 August 2024** | **13 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/019988**

| Patent document cited in search report | Publication date (day/month/year) | Patent family member(s) | Publication date (day/month/year) |
|---|---|---|---|
| WO 2023/054556 A1 | 06 April 2023 | (Family: none) | |
| WO 2022/084386 A1 | 28 April 2022 | JP 2023-546222 A | |
| WO 2020/003833 A1 | 02 January 2020 | US 2021/0147781 A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7097404 B
- WO 2023054556 A
- JP 2016517691 A
- JP 2000517188 A